(19) Europäisches Patentamt
European Patent Office
Office européen des brevets

(11) EP 2 734 839 B1

(12) **EUROPEAN PATENT SPECIFICATION**

(45) Date of publication and mention of the grant of the patent:
**08.11.2017 Bulletin 2017/45**

(51) Int Cl.:
**G01N 33/487** (2006.01)

(21) Application number: **12815126.3**

(22) Date of filing: **18.07.2012**

(86) International application number:
**PCT/US2012/047231**

(87) International publication number:
**WO 2013/012940 (24.01.2013 Gazette 2013/04)**

(54) **COMPENSATED PATCH-CLAMP AMPLIFIER FOR NANOPORE POLYNUCLEOTIDE SEQUENCING**

AUSBALANCIERTER PATCH-CLAMP-VERSTÄRKER ZUR POLYNUKLEOTID-NANOPOREN-SEQUENZIERUNG

AMPLIFICATEUR DE PATCH-CLAMP COMPENSÉ POUR LE SÉQUENÇAGE DE POLYNUCLÉOTIDES A L'AIDE D'UN NANOPORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2011 US 201161572829 P**

(43) Date of publication of application:
**28.05.2014 Bulletin 2014/22**

(73) Proprietor: **The Regents of the University of California**
**Oakland, CA 94607 (US)**

(72) Inventors:
• **DUNBAR, William**
**CA 95064 (US)**
• **KIM, Jungsuk**
**CA 95064 (US)**
• **PEDROTTI, Kenneth**
**CA 95064 (US)**

(74) Representative: **Boxall, Sarah Jane**
**Boxall IPM Ltd**
**9 King Street**
**Sandwich Kent CT13 9BT (GB)**

(56) References cited:
EP-A1- 1 271 144        WO-A2-00/71742
US-A1- 2006 194 255     US-B1- 6 528 258
US-B2- 7 741 829

• **GANG WANG ET AL: "An integrated, low noise patch-clamp amplifier for biological nanopore applications", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 August 2010 (2010-08-31), pages 2718-2721, XP031883396, DOI: 10.1109/IEMBS.2010.5626570 ISBN: 978-1-4244-4123-5**
• **JUNGSUK KIM ET AL: "An integrated patch-clamp amplifier for ultra-low current measurement on solid-state nanopore", SOC DESIGN CONFERENCE (ISOCC), 2010 INTERNATIONAL, IEEE, PISCATAWAY, NJ, USA, 22 November 2010 (2010-11-22), pages 424-427, XP032031189, DOI: 10.1109/SOCDC.2010.5682879 ISBN: 978-1-4244-8633-5**

EP 2 734 839 B1

## Description

## FIELD OF THE INVENTION

The presently disclosed subject matter is directed towards electronic devices and systems suitable for use in DNA sequencers and for detecting and quantifying individual nucleotides in a polynucleotide. More particularly, the present invention relates to a nanopore sequencer comprising a compensated patch-clamp amplifiers and their use in DNA sequencing systems and methods and in similar applications.

## BACKGROUND OF THE INVENTION

DNA was first isolated from cells by the Swiss scientist Friedrich Miescher in 1869. In 1944 Deoxyribonucleic Acid was discovered to be a chemical that comprised a tiny genetic encyclopedia in living cells. In 1953 James Watson, an American scientist, and Francis Crick, a British researcher working at the University of Cambridge in England discovered the now-famous "double helix" molecular structure of DNA for which they received a 1962 Nobel Prize.

In nanopore sequencing a DNA strand to be sequenced is passed through an ionic fluid filled sensor having a very small pore while a voltage is induced across the sensor. The resulting sensor current depends on the structure of the DNA strand. By analyzing the sensor current the DNA strand can be sequenced. While the theoretical framework of nanopore sequencing is well understood, prior art nanopore sequencing systems and devices were not fully developed. Nanopore sequencing currents are very small and any realistic nanopore sequencing system requires very high gains. Very high gains tend to create reading instabilities caused by distributed resistances and capacitances as well as internal and external noise.

Despite those problems the promise of nanopore sequencing has motivated the development of electronic devices and systems that can detect and quantify individual nucleotides in a polynucleotide. In practice a nanopore sensor has two chambers, referred to as a *cis* and a *trans* chamber. Those chambers are filled with a buffered ionic conducting solution (for example, KCl) and a voltage is applied across the nanopore chambers. As a result, a charged DNA initially placed in the *cis* chamber starts moving towards the *trans* side. As it traverses the nanopore, the ionic current momentarily decreases. The ionic current is typically in the range of tens to hundreds of picoAmperes. The resulting electric current depends on the number of ions (the charge/ net charge) in the nanopore as well as on the nanopore dimensions. The number and charge of ions can be the result of the DNA nucleotide strand passing through the nanopore (or approaching the nanopore opening). It is by monitoring the resulting current that the DNA nucleotide can be sequenced.

Accurately measuring the ultra-low current variations requires a very specialized amplifier that is referred to herein as a patch-clamp. Practical patch-clamps include an input headstage current-to-voltage converter and a difference amplifier that amplifies the voltage from the headstage. A patch-clamp must meet two very challenging design requirements. First, the input-offset voltage ($V_{OS}$) of the headstage must be minimized. Even the best high-gain amplifiers available have some $V_{OS}$. Causes for the $V_{OS}$ include random process mismatches and unavoidable systematic variations. Whatever the $V_{OS}$ is, it is amplified by the difference amplifier. In effect the $V_{OS}$ limits the output dynamic range.

Secondly, patch-clamp input parasitic capacitances have to be reduced to prevent headstage saturation. When a command voltage $V_{CMD}$ is applied to the nanopore sensor to produce operating currents, that voltage is actually applied through a resistance to an inverting input of an op-amp. Thus a command voltage $V_{CMD}$ change is time delayed due to unavoidable stray system capacitances. This causes a transient difference between the inverting input and the non-inverting input which leads to output saturation until the parasitic capacitances are charged and the inverting input once again is equal to $V_{CMD}$. During this interval, known as the 'deadtime' all incoming data is lost. Minimizing $V_{OS}$ and compensating for input parasitic capacitances and resistance are major design problems in nanopore sequencing.

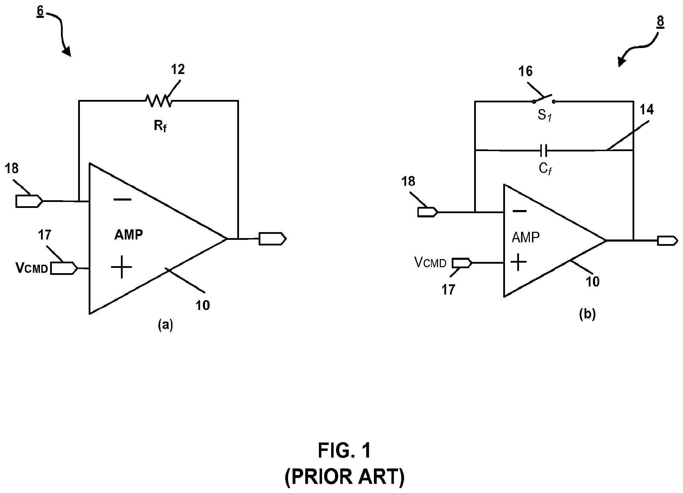

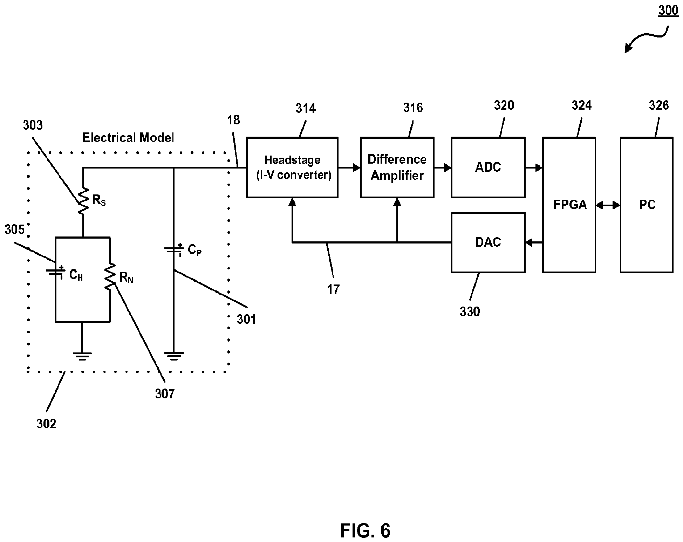

Modern patch-clamps are rather specialized high gain, differential op-amp transimpedance amplifiers that use either resistive or capacitive feedback. Figures 1(a) and 1(b) present those two basic patch-clamp architectures, as described by Wang and Dunbar (Proc. 32nd Annual International Conference of the IEEE EMBS (2010), pages 2718-2721). In any event, the basic patch-clamp comprises two components: an amplifier 10 and a compensation system that comprises either a resistor 12, reference the resistive feedback patch-clamp circuit 6 shown in Figure 1(a), or a capacitor 14 in parallel with a reset switch 16, reference the capacitive feedback patch-clamp circuit 8 shown in Figure 1(b). In both circuits a command voltage $V_{CMD}$ is applied to the non-inverting input 17 of the amplifier 10 while the potential across a nanopore sensor 302 (see for example Figure 6) is applied to the inverting input 18.

In Figure 1(a), the input current $I_{in}$ on the inverting input 18 is amplified in accord with the value of the feedback resistor 12 ($R_f$). The resulting transimpedance gain is simply $V_{OUT}=R_f \times I_{in}$. In Figure 1(b), the capacitive feedback acts as an integrator, and thus the amplifier 10 must in practice be followed by a differentiator.

In theory the basic patch-clamps 6 and 8 are sound. In practice, things go wrong. Transimpedance patch-clamp amplifiers that use resistive feedback, reference Figure 1(a), suffer from significant time delays following command voltage $V_{CMD}$ changes. Referencing the nanopore sensor 302 shown in Figure 6, those delays are a result of a pole formed by unavoidable stray capacitances ($C_P$) 301, the relatively large feedback resistor 12 (see Figure 1(a)), an unavoidable series resistance Rs 303, the nanopore sensor 302 capacitance ($C_N$) 305, and the nanopore sensor 302 resistance ($R_N$) 307. The resistive feedback patch-clamp circuit 6 shown in Figure 1(a) operates as a non-inverting amplifier with a gain of (1+$C_N$/$C_P$) just after the command voltage $V_{CMD}$ changes. Since $C_N$ is always larger than $C_P$ the output of the amplifier 10 becomes saturated and data is lost until the amplifier 10 has time to supply sufficient charge to the capacitances to allow a return to normal operation. That 'dead-time' is very undesirable.

In the prior art, complicated compensation circuitry has been used to attempt to avoid, shorten, or at least minimize dead-time. Such prior art compensation circuitry not only increased the complexity of the basic patch-clamp but resulted in an increased input capacitance which not only limited the bandwidth of resistive feedback patch-clamp circuits, such as the resistive feedback patch-clamp circuit 6, but usually resulted in output voltage "ringing" in response to a step input.

The capacitive feedback patch-clamp circuit 8 shown in Figure 1(b) was developed at least in part to avoid the dead-time and system complexity of resistive feedback patch-clamp circuits 6 (see Figure 1(a)). The capacitive feedback patch-clamp circuit 8 has a wide bandwidth and effectively a unity gain at the instant when the reset switch 16 is closed. By properly timing the closing of the reset switch 16 across the capacitor 14 having a capacitance of $C_f$, a command voltage $V_{CMD}$ change on the non-inverting terminal 17 does not initially affect the output of the amplifier 10 and output saturation is avoided.

Unfortunately, when the reset switch 16 opens, the input capacitance at the inverting input 18 increases by $C_f \times (1+A_0)$, wherein Ao is the gain of the amplifier 10, reference the well-known Miller's theorem. That rather dramatic input capacitance change subsequently restricts the bandwidth of the capacitive feedback patch-clamp circuit 8. Thus using capacitive feedback transimpedance amplifiers makes it very difficult to apply arbitrary command voltage $V_{CMD}$ changes because the reset frequency ($f_{RST}$) is determined by $I_{in}/(C_f \times \Delta V)$, where $\Delta V$ is the voltage difference between the inverting input 18 and the output voltage $V_O$. That frequency is not necessarily synchronized with command voltage $V_{CMD}$ changes.

One solution to the reset frequency- command voltage $V_{CMD}$ change problem is to simply increase the reset frequency ($f_{RST}$) by decreasing the capacitance $C_f$ of the capacitance 14 so that the reset frequency is compatible with the command voltage $V_{CMD}$ changes. This requires multiple capacitors and their proper selection as feedback capacitor 14 capacitances whenever waveforms having different transition periods are applied as the command voltage $V_{CMD}$ changes. The result is a much larger and more complex patch-clamp amplifier.

Prior art compensation of patch-clamp amplifiers used additional amplifiers to estimate series resistance ($R_S$) and parasitic capacitance ($C_P$), a rather complex circuit resulted.

Therefore, a nanopore sequencer comprising a new patch-clamp amplifier circuit that avoids the foregoing and other limitations in the prior art would be desirable. Even more desirable would be a nanopore sequencer comprising new patch-clamp amplifier systems that incorporate compensation tailored to the particular application. Ever more beneficial would be a nanopore sequencer comprising new patch-clamp systems having compensation that can be digitally controlled.

## BRIEF SUMMARY OF THE INVENTION

The principles of the present invention provide for techniques for a nanopore sequencer comprising patch-clamp amplifier circuits that incorporate compensation and that can be tailored to a particular application. The new nanopore sequencer comprising a patch-clamp circuit uses digitally controlled compensation and is used in a nanopore sequencer for sequencing polynucleotides, as defined by appended claim 1.

The nanopore sensor may comprise a semiconductive material or it may be a cell membrane. The patch-clamp circuit may include a current-to-voltage converter and a difference amplifier. The output is beneficially applied to an analog-to-digital converter that produces an amplified digital version of the current in the nanopore sensor. That amplified digital version can be input to a field programmable array and/or as an input to a computer. Preferably the computer operatively produces the timing signals and the timed digital command voltages.

## BRIEF DESCRIPTION OF THE DRAWINGS

The advantages and features of the present invention will become better understood with reference to the following detailed description and claims when taken in conjunction with the accompanying drawings, in which like elements are identified with like symbols, and in which:

Figure 1(a) is a schematic depiction of a prior art resistive feedback patch-clamp circuit;

Figure 1(b) is a depiction of a prior art capacitive feedback patch-clamp circuit;

Figure 2 is a schematic depiction of a simplified compensated patch-clamp circuit in accord with the principles of the present invention;

Figure 3(a) is a schematic depiction of the operation of the compensated patch-clamp circuit shown in Figure 2 when reset switch 16 is closed;

Figure 3(b) is a schematic depiction of the operation of the compensated patch-clamp circuit shown in Figure 2 when reset switch 16 is open;

Figure 4 is a schematic depiction of a compensated patch-clamp circuit in accord with the principles of the present invention that uses a digital-to-analog converter (DAC);

Figure 5 illustrates a schematic depiction of a prior art patch-clamp system and a nanopore sensor;

Figure 6 is a schematic depiction of a preferred embodiment compensated patch-clamp circuit;

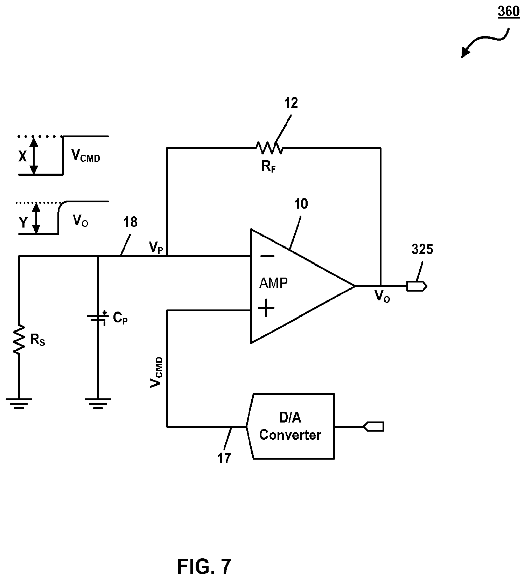

Figure 7 is a schematic depiction of a simplified version of the compensated patch-clamp circuit shown in Figure 6 during early resistor compensation operations;

Figure 8 is a schematic depiction of a simplified version of the compensated patch-clamp circuit shown in Figure 6 during later resistor compensation operations;

Figure 9 is an operational flow diagram for compensating nanopore sensor resistances;

Figure 10 is an operational flow diagram for compensating nanopore sensor capacitances;

Figure 11 is a schematic depiction of a simplified preferred embodiment compensated patch-clamp circuit during capacitor compensation; and

Figure 12 is a schematic depiction of a simplified preferred embodiment compensated capacitor patch-clamp circuit.

Figure 13 shows a three terminal nanopore sensor front end for practicing the present invention.

**DETAILED DESCRIPTION OF THE INVENTION**

The presently disclosed subject matter now will be described more fully hereinafter with reference to the accompanying drawings in which one embodiment is shown. However, it should be understood that this invention may take many different forms and thus should not be construed as being limited to the embodiment set forth herein.

In addition, in the figures like numbers refer to like elements throughout. Additionally, the terms "a" and "an" as used herein do not denote a limitation of quantity, but rather denote the presence of at least one of the referenced items.

In what follows a generic nanopore sensor 302 (reference Figure 6) is described, used, and compensated for. It should be understood that a nanopore sensor 302 might incorporate a living cellular membrane or it might incorporate a solid-state nanopore. Furthermore, while not all circuits that are subsequently described, specifically show a nanopore sensor 302, which is to better show the circuit operation, and thus it should be understood that a nanopore sensor 302 is, or can be, connected to the variously illustrated and described circuitry.

Devices suitable for use with the present invention are described in, for example, United States Patent Number (USPN) 5,795,782, USPN 6,015,714, USPN 6,267,872, USPN 6,627,067, USPN 6,746,594, USPN 6,428,959, USPN 6,617,113, and International Publication Number WO 2006/028508. Essentially while any individual device described herein may not be novel, the combination of the individual devices results in a new, useful, and non-obvious nanopore patch-clamp systems, DNA sequencers, and electrochemical applications for measuring biochemical analytic concentrations such as glucose, oxygen, neurotransmitters and pathogens that can be measured using transimpedance amplifiers or current-to-voltage converters.

Nanopore sensitivity, particularly in the case of solid-state nanopores, is determined by the pore size and the thickness. To identify a single nucleotide ($\approx$ 0.35nm) of single-stranded DNA in a nanopore sensor, the nanopore sensor will have a diameter of somewhere around 0.35nm or less. That causes a nanopore capacitance of about:

$$C = \varepsilon_r \varepsilon_0 \frac{A}{d},$$

where $\varepsilon_r$, $\varepsilon_0$, $A$ and $d$ indicate a relative permittivity, the electric constant ($8.854 \times 10^{-12}$ F m$^{-1}$), an exposed area, and thickness, respectively. Where atomic layers, i.e. $Al_2O_3$ and graphene, are used for the nanopore sensor the nanopore capacitance is larger, which results in longer dead-times (see below) when the command voltage changes. Such atomic layer sensors particularly benefit by the principles of the present invention.

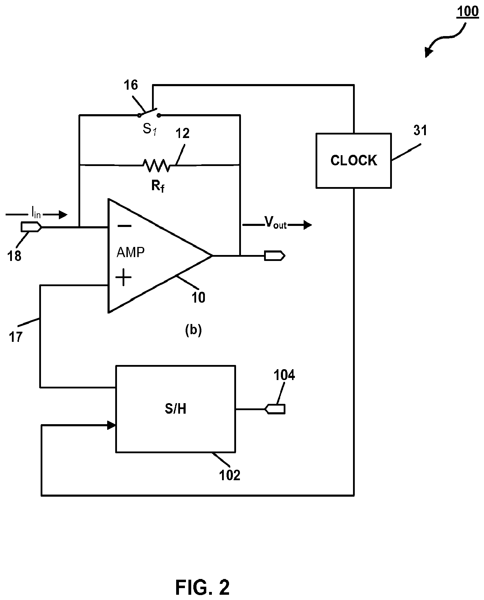

Figure 2 illustrates a basic compensated patch-clamp circuit 100 that is in accord with the present invention. The basic compensated patch-clamp circuit 100 differs in hardware from the resistive feedback patch-clamp circuit 6 (see Figure 1(a)) by the incorporation of a reset switch 16 for selectively shorting out the feedback resistor 12 and by the incorporation of a sample and hold circuit 102 that is disposed between the non-inverting input 18 and a command voltage $V_{CMD}$ applied to the input 104 of the sample and hold circuit 102.

During operation the reset switch 16 is closed in synchronization with step transitions of the output of the sample and hold circuit 102. In practice those transitions and the reset switch 16 synchronization are controlled by timing pulses from a clock 31. For purposes of clarity of explanation those timing pulses and the clock 31 are left out of subsequent figures. However it should be understood that the reset switch 16 operates in synchronization with command voltage $V_{CMD}$ changes, be they from a sample and hold circuit, a digital-to-analog converter, or some other circuit, and that some type of synchronized timing is required.

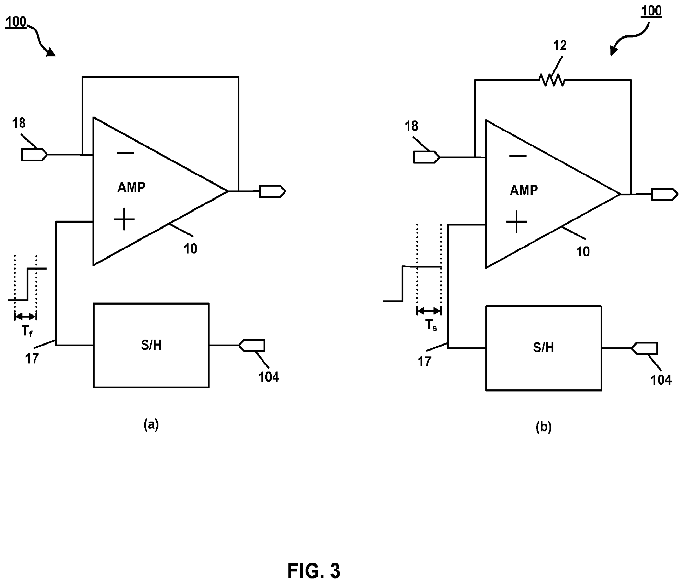

The basic compensated patch-clamp circuit 100 has two modes of operation: a transient mode when the command voltage $V_{CMD}$ changes, depicted in Figures 3(a), and a steady state mode when the command voltage $V_{CMD}$ is stable, depicted in Figure 3(b). In both operational modes it should be understood that the command voltage $V_{CMD}$ has been digitized into discrete steps. During transient mode operation the saturation and associated dead-time of the op-amp 10 is avoided by closing the reset switch 16. The operation of the compensated patch-clamp circuit 100 is then similar to the capacitive feedback pulse clamp circuit shown in Figure 1(b) and the op-amp 10 operates as a unity gain amplifier. In the steady-state mode the reset switch 16 is turned off and the basic compensated patch-clamp circuit 100 operates like the resistive-feedback patch-clamp shown in Figure 1(a).

Because a feedback capacitor 14 is not used in the basic compensated patch-clamp circuit 100 periodic reset pulses are not required to remove built up charges. Furthermore, complex compensation circuitry is also not required because resistive-feedback is used. The basic compensated patch-clamp circuit 100 architecture enables the use of complex command voltage $V_{CMD}$ waveforms and the use of various dwell times in addition to reduced hardware complexity.

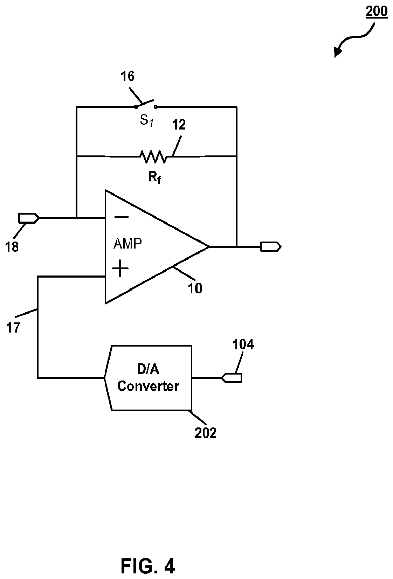

The basic compensated patch-clamp circuit 100 and its sample and hold circuit 102 represents a major change in nanopore patch-clamp circuits. One improvement to the basic compensated patch-clamp circuit 100 is shown in the improved compensated patch-clamp circuit 200 of Figure 4. The improved compensated patch-clamp circuits 200 uses a low-pass filtered digital-to-analog converter 202 in place of the sample and hold circuit 102 shown in Figure 2. The digital-to-analog converter 202 is an improvement because the digital-to-analog converter 202 can be directly connected to and controlled by a computerized system such as a personal computer. Such a computerized system is described subsequently; reference Figure 6 and its supporting description. In addition, the reset switch 16 can be controlled either by a computer or by a field programmable gate array. However, timing synchronization of the reset switch 16 operations and command voltage $V_{CMD}$ changes is still required, although the simple clock 31 shown in Figure 2 may be replaced by clocked digital-to-analog converter 202 timing signals or timing derived from the output of the computer.

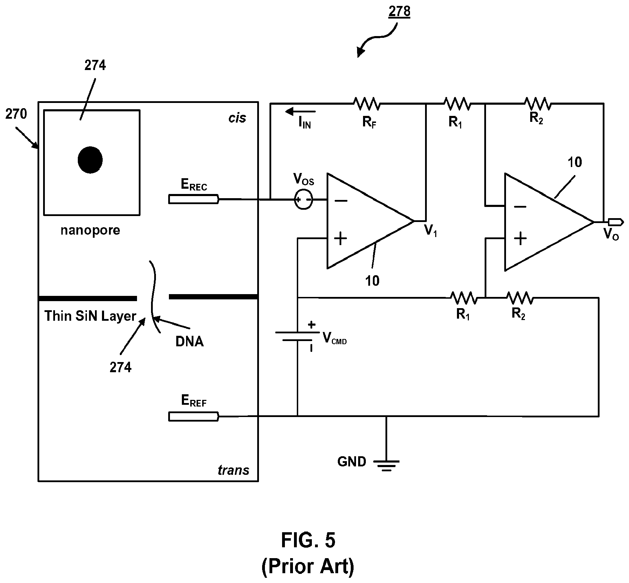

As noted patch-clamps have been used in prior art DNA sequencers. Figure 5 shows a prior art DNA sequencer 270. It comprises a nanopore sensor 272 having two "channels": a *cis* channel and a *trans* channel separated by a nanopore 274 through a semi-conductive material and retained in an ionic (KCl) fluid-filled container. The current that flows between the *cis* channel and the *trans* channel is converted by a first op-amp into a voltage (I-V conversion) and then amplified by difference amplifier. The basic patch-clamp amplifiers 6 and 8, reference Figure 1, in practice are replaced by a two-stage patch-clamp amplifier 278 having an I-V conversion stage and a difference amplifier stage.

While a nanopore sequencer comprising the basic patch-clamp circuits 100 and 200 are by themselves new, beneficial and useful, the preferred embodiment of the present invention is the computerized compensated DNA sequencer 300 system shown in Figure 6. The DNA sequencer 300 includes a nanopore sensor 302 which directly corresponds to the nanopore sensor 272 shown in Figure 5 except that the nanopore sensor 302 may comprise a cell membrane nanopore or a semi-conductive nanopore. For clarity of understanding Figure 6 presents an electrical model of the nanopore sensor 302 with the understanding that its physical configuration will be that of the nanopore sensor 272 or its cell membrane counterpart. That electrical model includes a nanopore capacitance 305 ($C_N$), a nanopore resistance ($R_N$) 307, an electrode series resistance ($R_S$) 303, and an input parasitic capacitance ($C_P$) 301.

The nanopore sensor 302 is connected to the inverting input 18 of a patch-clamp circuit comprised of an input (I-V) converter 314 headstage and a difference amplifier 316, which is analogous to that shown in Figure 5. The output of the patch-clamp circuit is input to an analog-to-digital converter 320 that digitizes its analog voltage input and applies its digitized output version as inputs to a field programmable gate array 324. The field programmable gate array 324 sends a suitably processed version of its received digitized voltage reading to a personal computer 326 (or another suitable computerized system).

The personal computer 326 performs data analysis on the nanopore sensor 302 reading. In addition, the personal computer PC 326 applies control signals to the field programmable gate array 324 which are subsequently used to control the operation of a digital-to-analog converter 330. The digital-to-analog converter 330 provides command voltages ($V_{CMD}$) to the non-inverting inputs 17 of the input (I-V) converter 314 headstage and the difference amplifier 316. Thus the operation of the DNA sequencer 300 is computer controlled, its outputs are available for data analysis, and patch-clamp compensation is provided as described below.

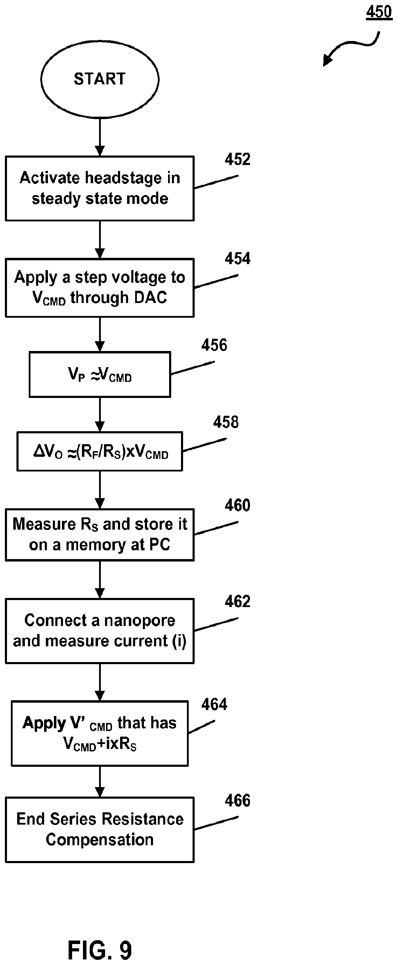

The DNA sequencer 300 is well suited for automated compensation. A compensation operation 450 is shown in the flow diagram of Figure 9. That operation 450 starts and proceeds by activating the input (I-V) converter 314 headstage and the difference amplifier 316 in a steady state mode, step 452. Obtaining a steady state mode is explained with the aid of a simplified patch-clamp circuit 360 (the input (I-V) converter 314 headstage and the difference amplifier 316) shown in Figure 6. Note that the simplified patch-clamp circuit 360 is shown without the nanopore sensor 302 and with the electrode series resistance ($R_S$) 303 and the input parasitic capacitance ($C_P$) 301 grounded. The series resistance ($R_S$) 303 and the parasitic capacitance ($C_P$) 301 are distributed and unavoidable. The command voltage ($V_{CMD}$) is set to a predetermined voltage (nominally ground). This causes the output voltage $V_O$ on the output terminal 325 to become stable and the patch-clamp circuit 360 is placed in a steady-state mode.

After some time a $V_{CMD}$ voltage step is applied, step 454 which, after some time delay, sets the voltage $V_P$ across the series resistance ($R_S$) 303 and the parasitic capacitance ($C_P$) 301 to $V_{CMD}$ see step 456. Next, the output voltage variation is measured, step 458. Note that the output voltage is digitized and applied to the PC 326. From the output voltage variation and from the known $R_F$ 12 the value of the electrode series resistance Rs can be accurately measured (determined), step 460. The formula relating the output voltage variation and Rs is shown in step 458.

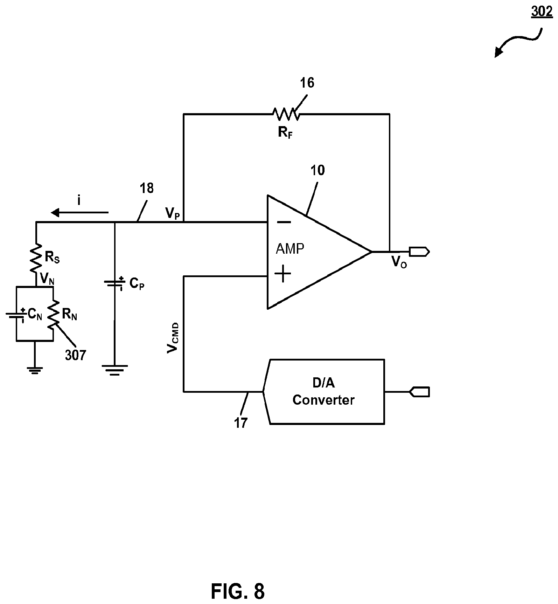

Next, a nanopore sensor 302 is applied to the patch-clamp amplifier 360 and the resulting nanopore current (i) is measured, step 462, reference Figure 8. After the current (i) is measured the PC 326 causes the digital-to-analog converter 330 via the field programmable gate array 324 to generate another, different command voltage $V'_{CMD}$ where $V'_{CMD} = V_{CMD} + i \times R_S$, step 464. This is possible because Rs was previously found (steps 450 through 460). The nanopore sensor 302 resistance $R_N$ 307 can also be determined from the output $V_O$ variation. Series resistance compensation is ended, step 466. Since all nanopore related resistances have been determined the actual voltage applied across the nanopore sensor 302 can accurately be known despite the series resistance ($R_S$) 303, the parasitic capacitance ($C_P$) 301 and the nanopore resistance 307. Thus the nanopore sensor 302 resistive environment is accurately compensated for.

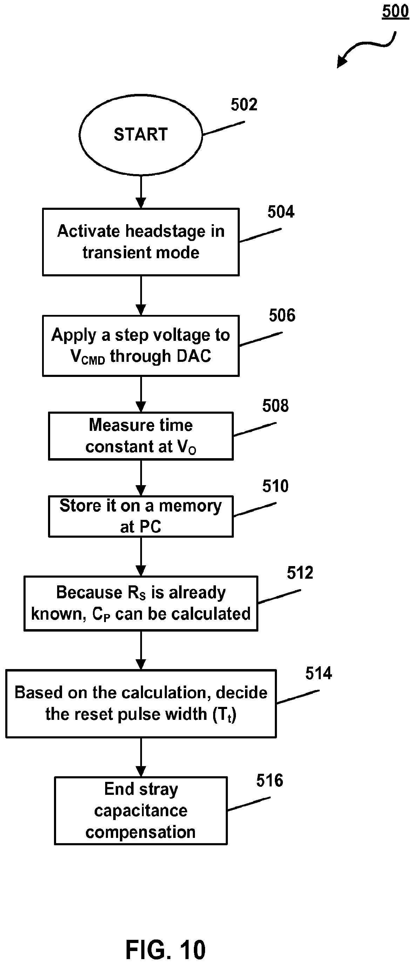

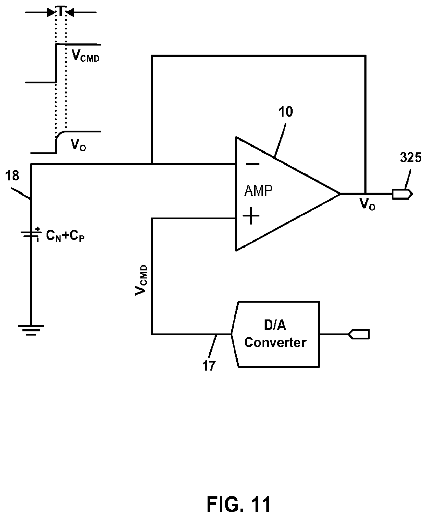

In addition to resistor compensation it is possible to compensate for capacitances. Figure 10 illustrates the operation 500 of capacitance compensation. The operation 500 starts, step 502 and proceeds by entering a transient mode, step 504. Figure 11 shows the transient mode which is entered by closing the reset switch 16 to short the inverting input to the output terminal 325, thus shorting out the feedback resistance $R_F$ 12, (see Figure 1(a)) and charging all capacitances. Next, a command voltage $V_{CMD}$ step is applied, step 506. The output voltage Vo on the output terminal 325 is monitored and the time constant of $V_O$ is measures, step 508, and stored in memory, step 510. Because the electrode series resistance $R_S$ and the time constant have been determined the value of the parasitic capacitor $C_P$, which is much smaller than the nanopore electrode capacitance $C_N$, can be accurately calculated, step 512. From the calculated value of $C_P$ a determination of an optimal reset pulse

width ($T_t$) can be decided, step 514. The reset pulse width should be somewhat longer than the time constant found in step 506 but should not be so long as to blank out the voltage step. By blank out it is meant that the reset pulse width is so long that the response of the patch-clamp circuit to the voltage step cannot be determined by the system before another step occurs. That reset pulse width delay compensates for the input parasitic capacitances including the inverting input electrode, the connecting cable, and the nanopore sensor and capacitor compensation ends, step 516.

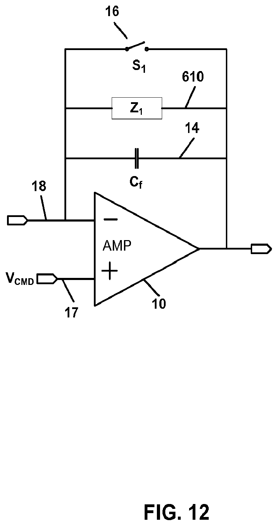

While the foregoing has described a novel nanopore sequencer comprising a resistive feedback patch-clamp system, its use in DNA sequencing, and automated compensation based on a resistive patch-clamp circuit, the principles of the present invention are also useful to a nanopore sequencer comprising capacitive patch-clamp circuits. Figure 12 helps illustrate how the compensation technique of the present invention can be applied to the capacitive-feedback transimpedance amplifiers. Periodic reset pulses are not required because of the high impedance $Z_1$ 610 caused by unavoidable leakage. By eliminating the periodic resets the glitch at the input due to charge and clock feed-through are avoided. However, $Z_1$ still requires compensation as does the parasitic input capacitance $C_P$ and the electrode series resistance $R_S$. By adding the reset switch 16 in parallel with $Z_1$ and $C_f$ 14 the compensation procedure for the capacitive-feedback TIA are the same as previously described.

Additional embodiments and disclosures are as follows.

The invention herein disclosed provides for devices and methods that can detect and quantify individual nucleotides in a polynucleotide. The device can be a solid-state nanopore or a nanopore positioned at a defined site, for example, upon a substrate and/or surface.

The devices herein disclosed can avoid 'dead-time' by placing a switch to a conventional transimpedance amplifier with a resistive feedback. Various discrete waveforms may be generated and applied to the voltage command by using a sample/hold circuit or DAC for the command voltage control. The voltage patch-clamp amplifier can be fully controlled by a computer interface system.

The invention also discloses a method of compensating for the feedback resistors as disclosed above. The invention further discloses a method for compensating for the probe input capacitance.

The invention can be used to detect the position and measure the quantity of a molecule relative to the nanopore. The molecule can be positioned by varying the potential difference on either side of the nanopore. The molecule can be a macromolecule and can further comprise a polyion, such as a polyanion and/or a polycation. In one a preferred embodiment, the polyion is a polynucleotide. In another preferred embodiment the polyion is a polypeptide. The substrate and/or surface can delimit two chambers and can further comprise a pore, the pore located at the substrate or surface. One of the chambers is *cis* to the pore and the other chamber is *trans* to the pore. The molecule can be positioned by varying the potential difference between the chambers. Preferably, the molecule is initially present in the *cis* chamber. The presence and/or absence and/or change in the molecular composition can be detected by measuring the electric current through the pore. The invention can be used as a sensor that detects molecules. The invention is of particular use in the fields of molecular biology, structural biology, cell biology, molecular switches, molecular circuits, and molecular computational devices, and the manufacture thereof.

The nanopore device systems may comprise *'cis'* and *'trans'* chambers connected by an electrical communication means. In one embodiment the chambers comprise a medium, the medium selected from the group consisting of an aqueous medium, a non-aqueous medium, an organic medium, or the like. In one embodiment the medium is a fluid. In an alternative embodiment the medium is a gas. In one embodiment the electrical communication means is a solid state pore comprising, for example, silicon nitride, bifunctional alkyl sulfide, and/or gold or other metal or alloy. In the alternative, the *cis* and *trans* chambers are separated by a thin film comprising at least one pore or channel. In one preferred embodiment, the thin film comprises a compound having a hydrophobic domain and a hydrophilic domain. In a more preferred embodiment, the thin film comprises a phospholipid. The devices further comprise a means for applying an electric field between the *cis* and the *trans* chambers. In one embodiment the pore or channel accommodates a part of the polyion. In another embodiment the pore or channel accommodates a part of the molecule. In one preferred embodiment, the molecule is a macromolecule. In another preferred embodiment the polyion is selected from the group consisting of polynucleotides, polypeptides, phospholipids, polysaccharides, and polyketides.

In one embodiment the compound comprises an enzyme. The enzyme activity can be, for example, but not limited to, enzyme activity of proteases, kinases, phosphatases, hydrolases, oxidoreductases, isomerases, transferases, methylases, acetylases, ligases, lyases, ribozyme, and the like. In a more preferred embodiment the enzyme activity can be enzyme activity of DNA polymerase, RNA polymerase, endonuclease, exonuclease, DNA ligase, DNase, uracil-DNA glycosidase, kinase, phosphatase, methylase, acetylase, glucose oxidase, ribozyme, and the like.

In still a further interesting embodiment, the pore is sized and shaped to allow passage of an activator, wherein the activator is selected from the group consisting of ATP, NAD$^+$, NADP$^+$, diacylglycerol, phosphatidylserine, eicosinoids, retinoic acid, calciferol, ascorbic acid, neuropeptides, enkephalins, endorphins, 4-aminobutyrate (GABA), 5-hydroxytryptamine (5-HT), catecholamines, acetyl CoA, S-adenosylmethionine, hexose sugars, pentose sugars, phospholipids, lipids, glycosyl phosphatidyl inositols (GPIs), and any other biological activator.

In certain embodiments the pore is sized and shaped to allow passage of a monomer, wherein the monomer is selected from the group consisting of dATP, dGTP, dCTP, dTTP, UTP, alanine, cysteine, aspartic acid, glutamic acid, phenylalanine, glycine, histidine, isoleucine, lysine, leucine, methionine, asparagines, proline, glutamine, arginine, serine, threonine, valine, tryptophan, tyrosine, hexose sugars, pentose sugars, phospholipids, lipds, and any other biological monomer.

In yet another embodiment the pore is sized and shaped to allow passage of a cofactor, wherein the cofactor is selected from the group consisting of Mg$^{2+}$, Mn$^{2+}$, Ca$^{2+}$, ATP, NAD$^+$, NADP$^+$, and any other biological cofactor.

In one important embodiment, the pore or channel comprises a biological molecule, or a synthetic modified or altered biological molecule. Such biological molecules are, for example, but not limited to, an ion channel, such as a-hemolysin, a nucleoside channel, a peptide channel, a sugar transporter, a synaptic channel, a transmembrane receptor, such as GPCRs, a receptor tyrosine kinase, and the like, a T-cell receptor, an MHC receptor, a nuclear receptor, such as a steroid hormone receptor, a nuclear pore, or the like.

In an alternative, the compound comprises a non-enzyme biological activity. The compound having non-enzyme biological activity can be, for example, but not limited to, proteins, peptides, antibodies, antigens, nucleic acids, peptide nucleic acids (PNAs), locked nucleic acids (LNAs), morpholinos, sugars, lipids, glycosyl phosphatidyl inositols, glycophosphoinositols, lipopolysaccharides, or the like. The compound can have antigenic activity. The compound can have ribozyme activity. The compound can have selective binding properties whereby the polymer binds to the compound under a particular controlled environmental condition, but not when the environmental conditions are changed. Such conditions can be, for example, but not limited to, change in [H$^+$], change in environmental temperature, change in stringency, change in hydrophobicity, change in hydrophilicity, or the like.

In one embodiment the macromolecule comprises an enzyme activity. The enzyme activity can be, for example, but not limited to, enzyme activity of proteases, kinases, phosphatases, hydrolases, oxidoreductases, isomerases, transferases, methylases, acetylases, ligases, lyases, and the like. In a more preferred embodiment the enzyme activity can be enzyme activity of DNA polymerase, RNA polymerase, endonuclease, exonuclease, DNA ligase, DNase, uracil-DNA glycosidase, kinase, phosphatase, methylase, acetylase, glucose oxidase, or the like. In an alternative embodiment, the macromolecule can comprise more that one enzyme activity, for example, the enzyme activity of a cytochrome P450 enzyme. In another alternative embodiment, the macromolecule can comprise more than one type of enzyme activity, for example, mammalian fatty acid synthase. In another embodiment the macromolecule comprises a ribozyme activity.

It is to be understood that while the figures and the above description illustrates the present invention, they are exemplary only. They are not intended to be exhaustive or to limit the invention to the precise forms disclosed, and obviously many modifications and variations are possible in light of the above teaching. Others who are skilled in the applicable arts will recognize numerous modifications and adaptations of the illustrated embodiments that remain within the principles of the present invention. Therefore, the present invention is to be limited only by the appended claims.

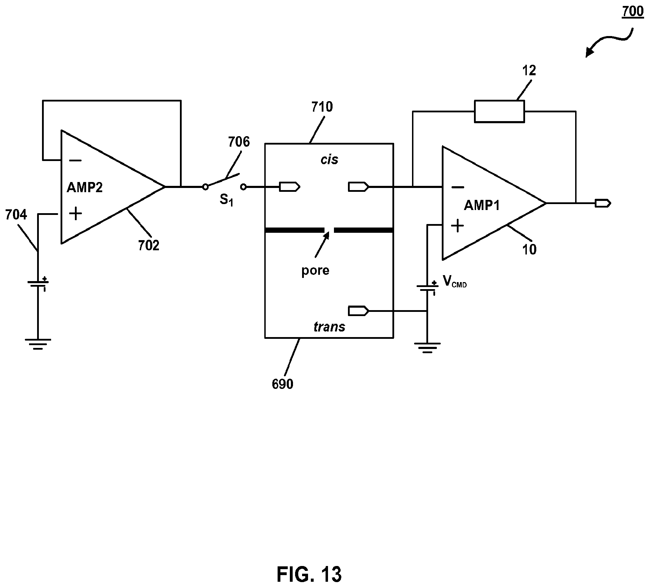

While the foregoing has explained the present invention using traditional two-electrode nanopore sensors the principles of the present invention are flexible enough to be used with other architectures. For example, Figure 13 shows a three electrode nanopore sensor 690 front end circuit 700. A unity-gain buffer amplifier 702 buffers the command voltage VCMD on its non-inverting input. Its buffered output is connected to the cis chamber through a switch S1 706. When the command voltage VCMD is changed the switch S1 706 turns on to inject current to charge the nanopore sensor's capacitance CN until the cis chamber 710 potential equals VCMD. This assists compensating for dead times. The compensation technique invented here can be applied to nanopore application, patch-clamp application and electrochemical applications to measure biochemical analytic concentrations, such as glucose, oxygen, neurotransmitters and pathogens that can be measured using a transimpedance amplifier or a current-to-voltage converter.

## Claims

1. A nanopore sequencer (300) for sequencing polynucleotides comprising a patch-clamp circuit (100, 200, 360) a nanopore sensor (302) and a computer (326), wherein the patch clamp circuit (100, 200, 360) comprises:

   a) a circuit (31) configured to produce timing signals;
   b) a differential amplifier circuit (10) comprising a non-inverting input (17), an inverting input (18) having a parasitic capacitance $C_P$ (301) and being connected to a first end of an electrode having an electrode series resistance $R_E$, an output (325), a feedback resistor (12) connected between said output and said inverting input (18), and a reset switch (16) that is configured to receive said timing signals and, in response to said timing signals, is configured selectively to connect said output (325) to said inverting input (18);

   c) a command voltage circuit (102, 202) configured to receive timing signals and command voltages, said command voltage circuit configured to apply stepped command voltages to said non-inverting input (17) in response to said timing signals; wherein the nanopore sensor (302) has an input capacitance $C_N$ (305), an input resistance $R_N$ (307), and a series resistance $R_S$ (303);

   **characterized in that** the computer (326) is configured to control the following steps:

   i) connect the second end of the electrode to ground and obtain a steady state output of the patch-clamp circuit (100, 200, 360) by setting a voltage on the non-inverting input (17) of the differential amplifier circuit to a reference voltage;
   ii) apply a step voltage to the non-inverting input (17);
   iii) determine the output voltage variation of the patch-clamp circuit (100, 200, 360) in response to the step voltage;
   iv) calculate the electrode series resistance $R_E$ using the output voltage variation determined in step iii);
   v) connect the nanopore sensor (302) between the second end of the electrode and ground and obtain a steady state output of the patch-clamp circuit (100, 200, 360) by setting the non-inverting input (17) to a reference voltage;
   vii) measuring the sensor current i after the steady state output is achieved; and
   viii) determining the sensor series resistance Rs (303) from the measured sensor current i, the electrode series resistance $R_E$, and the steady state output, and further configured to control the steps of:

   closing said reset switch (16) in synchronization with a step change in said stepped command voltages for a time TR; opening said reset switch (16) after said time TR; setting said time TR to prevent saturation of said differential amplifier circuit without blanking said stepped voltage; and selecting said stepped command voltages to produce a predetermined voltage across said nanopore sensor (302), by applying a compensated voltage to the non-inverting input (17) where the compensated voltage is equal to the predetermined voltage plus the sensor current i times the sensor series resistance $R_S$ (303).

2. The nanopore sequencer according to claim 1, wherein the nanopore sensor (302) includes a nanopore with a diameter of 0.35nm or less.

**3.** The nanopore sequencer according to claim 1 or claim 2, wherein said differential amplifier circuit includes a current-to-voltage converter (314) and a difference amplifier (316).

**4.** The nanopore sequencer according to any one of claims 1 to 3, wherein said command voltage circuit comprises a sample and hold circuit (102).

**5.** The nanopore sequencer according to any one of claims 1 to 3, wherein said command voltage circuit comprises a digital-to-analog converter (202, 330).

**6.** The nanopore sequencer according to claim 5, wherein said output is applied to an analog-to-digital converter (320) that produces an amplified digital version of said current in said nanopore sensor (302).

**7.** The nanopore sequencer according to claim 6, wherein said amplified digital version is applied to a field programmable array (324).

**8.** The nanopore sequencer according to claim 7, wherein said amplified digital version is input to the computer (326) and wherein said computer (326) causes said command voltages to be applied to said command voltage circuit.

**9.** The nanopore sequencer according to claims 1-8, wherein said nanopore sensor (302) comprises a semi-conductive material.

**10.** The nanopore sequencer according to claims 1-8, wherein said nanopore sensor (302) comprises a cell membrane.

**11.** The nanopore sequencer according to any one of claims 1-10, adapted to sequence a polynucleotide.

**12.** A method for using a nanopore sequencer as claimed in any one of claims 1 to 11, comprising the steps of:

 a) connecting a first end of an electrode to an inverting input (18) of a patch-clamp circuit (100, 200, 360);
 b) connecting a second end of the electrode to ground;
 c) connecting a feedback resistor $R_F$ (12) between the inverting input (18) and the output (325) of the patch-clamp circuit (100, 200, 360);
 d) obtaining a steady state output of the patch-clamp circuit (100, 200, 360) by setting the voltage on the non-inverting input (17) to a reference voltage;
 e) applying a step voltage to the non-inverting input (17);

 f) determining a variation in the output voltage of the patch-clamp circuit (100, 200, 360) in response to the step voltage;
 g) calculating the electrode series resistance $R_E$ using the output voltage variation determined in step f);
 h) connecting a nanopore sensor (302) between the second end of the electrode and ground;
 i) obtaining a steady state output of the patch-clamp circuit (100, 200, 360) by setting the non-inverting input (17) to a reference voltage;
 j) measuring the sensor current i after the steady state output is achieved;
 k) determining the sensor series resistance $R_S$ (303) from the measured sensor current i, the electrode series resistance $R_E$, and the steady state output;
 l) obtaining a predetermined voltage across the nanopore sensor (302) by applying a compensated voltage to the non-inverting input (17) where the compensated voltage is equal to the predetermined voltage plus the sensor current i times the sensor series resistance $R_S$ (303).

**13.** A method according to claim 12, further including the steps:

 activating the patch-clamp circuit (100, 200, 360) to achieve a steady state response after the sensor series resistance $R_S$ (303) has been determined;
 applying a compensation step voltage to a non-inverting input (17) of the patch-clamp circuit (100, 200, 360);
 determining the time constant of the output (325) of the patch-clamp circuit (100, 200, 360) to the compensation step voltage; and
 determining the input parasitic capacitance $C_P$ (301) of the nanopore sensor (302) from the sensor series resistance $R_S$ (303) and the determined time constant.

**14.** A method according to claim 13, further including the step of determining a reset pulse width based on the determined input parasitic capacitance (301).


**Patentansprüche**

**1.** Nanopore-Sequenzer (300) zum Sequenzieren von Polynukleotiden, aufweisend eine Patch-Clamp-Schaltung (100, 200, 360), einen Nanopore-Sensor (302) und einen Computer (326), wobei die Patch-Clamp-Schaltung (100, 200, 360) aufweist:

 a) eine Schaltung (301), die konfiguriert ist, Zeitsteuersignale zu erzeugen;
 b) eine Differenzverstärkerschaltung (10), aufweisend einen nicht invertierenden Eingang (17), einen invertierenden Eingang (18) mit einer parasitären Kapazität $C_P$ (301) und der mit einem ersten Ende einer Elektrode mit einem Elektrodenreihenwiderstand $R_E$ verbunden ist, einen Ausgang (325), einen Rückkopplungswiderstand (12), der zwischen dem Ausgang und dem invertierenden Eingang (18) angeschlossen ist, und einen Rücksetzschalter (16), der konfiguriert ist, die Zeitsteuersignale zu empfangen, und, in Reaktion auf die Zeitsteuersignale, selektiv konfiguriert ist, den Ausgang (325) mit dem invertierenden Eingang (18) zu verbinden; c) eine Befehlsspannungsschaltung (102, 202), die konfiguriert ist, Zeitsteuersignale und Befehlsspannungen zu empfangen, wobei die Befehlsspannungsschaltung konfiguriert ist, als Reaktion auf die Zeitsteuersignale gestufte Befehlsspannungen an dem nicht invertierenden Eingang (17) anzulegen;

wobei der Nanopore-Sensor (302) eine Eingangskapazität $C_N$ (305), einen Eingangswiderstand $R_N$ (307), und einen Serienwiderstand $R_S$ (303) aufweist;

**dadurch gekennzeichnet, dass**

der Computer (326) konfiguriert ist, die folgenden Schritte zu steuern:

i) Verbinden des zweiten Endes der Elektrode mit Masse und Erhalten eines stationären Ausgangs der Patch-Clamp-Schaltung (100, 200, 360) durch Einstellen einer Spannung an dem nicht invertierenden Eingang (17) der Differenzverstärkerschaltung auf eine Referenzspannung;

ii) Anlegen einer Stufenspannung an dem nicht invertierenden Eingang (17);

iii) Bestimmen der Ausgangsspannungsänderung der Patch-Clamp-Schaltung (100, 200, 360) als Reaktion auf die Stufenspannung;

iv) Berechnen des Elektrodenreihenwiderstands $R_E$ unter Verwendung der in Schritt iii) bestimmten Ausgangsspannungsvariation;

v) Verbinden des Nanopore-Sensors (302) zwischen dem zweiten Ende der Elektrode und der Masse und Erhalten eines stationären Ausgangs der Patch-Clamp-Schaltung (100, 200, 360), durch Einstellen des nicht invertierenden Eingangs (17) auf eine Referenzspannung;

vii) Messen des Sensorstroms i nachdem der stationäre Ausgang erreicht ist; und

viii) Bestimmen des Sensorreihenwiderstandes $R_S$ (303) aus dem gemessenen Sensorstrom i, des Elektrodenreihenwiderstands $R_E$ und dem stationären Ausgang,

und die ferner konfiguriert ist, die folgenden Schritte zu steuern von:

Schließen des Rücksetzschalters (16) synchron mit einer Stufenänderung in den gestuften Befehlsspannungen für eine Zeit TR;

Öffnen des Rücksetzschalters (16) nach der Zeit TR;

Einstellen der Zeit TR, um eine Sättigung der Differenzverstärkerschaltung zu verhindern, ohne die gestufte Spannung zu löschen; und

Auswählen der gestuften Befehlsspannungen, um eine vorbestimmte Spannung über dem Nanopore-Sensor (302) zu erzeugen, durch Anlegen einer kompensierten Spannung an dem nicht invertierenden Eingang (17), wobei die kompensierte Spannung gleich der vorbestimmten Spannung plus dem Sensorstrom i mal dem Sensorreihenwiderstand $R_S$ (303) ist.

2. Nanopore-Sequenzer nach Anspruch 1, wobei der Nanopore-Sensor (302) eine Nanopore mit einem Durchmesser von 0,35 nm oder weniger enthält.

3. Nanopore-Sequenzer nach Anspruch 1 oder Anspruch 2, wobei die Differenzverstärkerschaltung einen Strom-Spannungs-Wandler (314) und einen Differenzverstärker (316) enthält.

4. Nanopore-Sequenzer nach einem der Ansprüche 1 bis 3, wobei die Befehlsspannungsschaltung eine Abtast- und Halteschaltung (102) aufweist.

5. Nanopore-Sequenzer nach einem der Ansprüche 1 bis 3, wobei die Befehlsspannungsschaltung einen Digital-Analog-Wandler (202, 330) aufweist.

6. Nanopore-Sequenzer nach Anspruch 5, wobei der Ausgang an einem Analog-Digital-Wandler (320) angelegt wird, der eine verstärkte digitale Version des Stroms in dem Nanopore-Sensor (302) erzeugt.

7. Nanopore-Sequenzer nach Anspruch 6, wobei die verstärkte digitale Version auf ein feldprogrammierbares Array (324) angelegt wird.

8. Nanopore-Sequenzer nach Anspruch 7, wobei die verstärkte digitale Version in den Computer (326) eingegeben wird und wobei der Computer (326) verursacht, dass die Befehlsspannungen an die Befehlsspannungsschaltung angelegt werden.

9. Nanopore-Sequenzer nach einem der Ansprüche 1 bis 8, wobei der Nanopore-Sensor (302) ein halbleitendes Material aufweist.

10. Nanopore-Sequenzer nach einem der Ansprüche 1 bis 8, wobei der Nanopore-Sensor (302) eine Zellmembran aufweist.

**11.** Nanopore-Sequenzer nach einem der Ansprüche 1 bis 10, der zum Sequenzieren eines Polynukleotids geeignet ist.

**12.** Verfahren zur Verwendung eines Nanopore-Sequenzers nach einem der Ansprüche 1 bis 11, aufweisend die Schritte von:

a) Verbinden eines ersten Endes einer Elektrode mit einem invertierenden Eingang (18) einer Patch-Clamp-Schaltung (100, 200, 360);
b) Verbinden eines zweiten Endes der Elektrode mit Masse;
c) Verbinden eines Rückkopplungswiderstands $R_F$ (12) zwischen dem invertierenden Eingang (18) und dem Ausgang (325) der Patch-Clamp-Schaltung (100, 200, 360);
d) Erhalten eines stationären Ausgangs der Patch-Clamp-Schaltung (100, 200, 360) durch Einstellen der Spannung an dem nicht invertierenden Eingang (17) auf eine Referenzspannung;
e) Anlegen einer Stufenspannung an dem nicht invertierenden Eingang (17);
f) Bestimmen einer Änderung in der Ausgangsspannung der Patch-Clamp-Schaltung (100, 200, 360) als Reaktion auf die Stufenspannung;
g) Berechnen des Elektrodenreihenwiderstands $R_E$ unter Verwendung der in Schritt f) ermittelten Ausgangsspannungsänderung;
h) Verbinden eines Nanopore-Sensors (302) zwischen dem zweiten Ende der Elektrode und Masse;
i) Erhalten eines stationären Ausgangs der Patch-Clamp-Schaltung (100, 200, 360) durch Einstellen des nicht invertierenden Eingangs (17) auf eine Referenzspannung;
j) Messen des Sensorstroms i nachdem der stationäre Ausgang erreicht ist;
k) Bestimmen des Sensorreihenwiderstandes $R_S$ (303) aus dem gemessenen Sensorstrom i, des Elektrodenreihenwiderstands $R_E$ und des stationären Ausgangs;
l) Erhalten einer vorbestimmten Spannung über dem Nanopore-Sensor (302) durch Anlegen einer kompensierten Spannung an dem nicht invertierenden Eingang (17), wobei die kompensierte Spannung gleich der vorbestimmten Spannung plus dem Sensorstrom i mal dem Sensorreihenwiderstand $R_S$ (303) ist.

**13.** Verfahren nach Anspruch 12, ferner enthaltend die Schritte:

Aktivieren der Patch-Clamp-Schaltung (100, 200, 360), um eine stationäre Reaktion zu erreichen, nachdem der Sensorreihenwiderstand $R_S$ (303) bestimmt worden ist;

Anlegen einer Kompensationsstufenspannung an einem nicht invertierenden Eingang (17) der Patch-Clamp-Schaltung (100, 200, 360);
Bestimmen der Zeitkonstante des Ausgangs (325) der Patch-Clamp-Schaltung (100, 200, 360) an die Kompensationsstufenspannung; und
Bestimmen der eingegebenen parasitären Kapazität $C_P$ (301) des Nanopore-Sensors (302) aus dem Sensorreihenwiderstand $R_S$ (303) und der bestimmten Zeitkonstante.

**14.** Verfahren nach Anspruch 13,

ferner enthaltend den Schritt des Bestimmens einer Rückstellpulsbreite auf Basis der bestimmten eingegebenen parasitären Kapazität (301).

**Revendications**

**1.** Séquenceur de nanopore (300) pour séquencer des polynucléotides comprenant un circuit patch-clamp (100, 200, 360), un capteur à nanopore (302) et un ordinateur (326), dans lequel le circuit patch-clamp (100, 200, 360) comprend :

a) un circuit (31) configuré pour produire des signaux de synchronisation ;
b) un circuit d'amplificateur différentiel (10) comprenant une entrée non inverseuse (17), une entrée inverseuse (18) ayant une capacité parasite $C_P$ (301) et connectée à une première extrémité d'une électrode ayant une résistance en série d'électrode $R_E$, une sortie (325), une résistance de rétroaction (12) connectée entre ladite sortie et ladite entrée inverseuse (18), et un commutateur de remise à zéro (16) qui est configuré pour recevoir lesdits signaux de synchronisation et, en réponse auxdits signaux de synchronisation, est configuré pour connecter sélectivement ladite sortie (325) à ladite entrée inverseuse (18) ;
c) un circuit de tension de commande (102, 202) configuré pour recevoir des signaux de synchronisation et des tensions de commande, ledit circuit de tension de commande étant configuré pour appliquer des tensions de commande par paliers à ladite entrée non inverseuse (17) en réponse aux signaux de synchronisation,

dans lequel le capteur à nanopore (302) a une capacité d'entrée $C_N$ (305), une résistance d'entrée $R_N$ (307) et une résistance en série $R_S$ (303) ;
**caractérisé en ce que** :

l'ordinateur (326) est configuré pour commander les étapes suivantes :

i) connecter la seconde extrémité de l'électrode à la terre et obtenir une sortie d'état permanent du circuit patch-clamp (100, 200, 360) en réglant une tension sur l'entrée non inverseuse (17) du circuit d'amplificateur différentiel à une tension de référence ;
ii) appliquer une tension de palier à l'entrée non inverseuse (17) ;
iii) déterminer la variation de tension de sortie du circuit patch-clamp (100, 200, 360) en réponse à la tension de palier ;
iv) calculer la résistance en série d'électrode $R_E$ en utilisant la variation de tension de sortie déterminée à l'étape iii) ;
v) connecter le capteur à nanopore (302) entre la seconde extrémité de l'électrode et la terre et obtenir une sortie d'état permanent du circuit patch-clamp (100, 200, 360) en réglant l'entrée non inverseuse (17) à une tension de référence ;
vii) mesurer le courant de capteur i une fois que la sortie d'état permanent est atteinte ; et
viii) déterminer la résistance en série $R_S$ (303) du capteur à partir du courant mesuré i du capteur, de la résistance en série $R_E$ de l'électrode et de la sortie d'état permanent,

et configuré en outre pour commander les étapes consistant à :

fermer ledit commutateur de remise à zéro (16) en synchronisation avec un changement de palier dans lesdites tensions de commande par palier pendant une période TR ;
ouvrir ledit commutateur de remise à zéro (16) après ladite période TR ; régler ladite période TR pour empêcher la saturation dudit circuit d'amplificateur différentiel sans supprimer ladite tension de palier ; et sélectionner lesdites tensions de commande par palier pour produire une tension prédéterminée aux bornes dudit capteur à nanopore (302) en appliquant une tension compensée à l'entrée non inverseuse (17), où la tension compensée est égale à la tension prédéterminée plus le courant de capteur i fois la résistance en série $R_S$ (303) du capteur.

2. Séquenceur de nanopore selon la revendication 1, dans lequel le capteur à nanopore (302) comprend un nanopore d'un diamètre de 0,35 nm ou moins.

3. Séquenceur de nanopore selon la revendication 1 ou 2, dans lequel ledit circuit d'amplificateur différentiel comprend un convertisseur courant-tension (314) et un amplificateur de différence (316).

4. Séquenceur de nanopore selon l'une quelconque des revendications 1 à 3, dans lequel ledit circuit de tension de commande comprend un circuit échantillonneur-bloqueur (102).

5. Séquenceur de nanopore selon l'une quelconque des revendications 1 à 3, dans lequel ledit circuit de tension de commande comprend un convertisseur numérique-analogique (202, 330).

6. Séquenceur de nanopore selon la revendication 5, dans lequel ladite sortie est appliquée à un convertisseur analogique-numérique (320) qui produit une version numérique amplifiée dudit courant dans ledit capteur à nanopore (302).

7. Séquenceur de nanopore selon la revendication 6, dans lequel ladite version numérique amplifiée est appliquée à un réseau logique programmable par l'usager (324).

8. Séquenceur de nanopore selon la revendication 7, dans lequel ladite version numérique amplifiée est saisie dans l'ordinateur (326) et dans lequel ledit ordinateur (326) amène lesdites tensions de commande à s'appliquer audit circuit de tension de commande.

9. Séquenceur de nanopore selon les revendications 1 à 8, dans lequel ledit capteur à nanopore (302) comprend un matériau semi-conducteur.

10. Séquenceur de nanopore selon les revendications 1 à 8, dans lequel ledit capteur à nanopore (302) comprend une membrane cellulaire.

11. Séquenceur de nanopore selon l'une quelconque des revendications 1 à 10, adapté pour séquencer un polynucléotide.

12. Procédé d'utilisation d'un séquenceur de nanopore selon l'une quelconque des revendications 1 à 11, comprenant les étapes consistant à :

a) connecter une première extrémité d'une électrode à une entrée inverseuse (18) d'un circuit patch-clamp (100, 200, 360) ;
b) connecter une seconde extrémité de l'électrode à la terre ;
c) connecter une résistance de rétroaction $R_F$ (12) entre l'entrée inverseuse (18) et la sortie (325) du circuit patch-clamp (100, 200, 360) ;
d) obtenir une sortie d'état permanent du circuit patch-clamp (100, 200, 360) en réglant la tension sur l'entrée non inverseuse (17) à une tension de référence ;

e) appliquer une tension de palier à l'entrée non inverseuse (17) ;

f) déterminer une variation de la tension de sortie du circuit patch-clamp (100, 200, 360) en réponse à la tension de palier ;

g) calculer la résistance en série $R_E$ de l'électrode en utilisant la variation de tension de sortie déterminée à l'étape f) ;

h) connecter un capteur à nanopore (302) entre la seconde extrémité de l'électrode et la terre ;

i) obtenir une sortie d'état permanent du circuit patch-clamp (100, 200, 360) en réglant l'entrée non inverseuse (17) à une tension de référence ;

j) mesurer le courant i du capteur une fois que la sortie d'état permanent est atteinte ;

k) déterminer la résistance en série $R_S$ (303) du capteur à partir du courant de capteur mesuré i, de la résistance en série $R_E$ de l'électrode et de la sortie d'état permanent ;

l) obtenir une tension prédéterminée aux bornes du capteur à nanopore (302) en appliquant une tension compensée à l'entrée non inverseuse (17) où la tension compensée est égale à la tension prédéterminée plus le courant i du capteur fois la résistance en série $R_S$ (303) du capteur.

**13.** Procédé selon la revendication 12 comprenant en outre les étapes consistant à :

activer le circuit patch-clamp (100, 200, 360) pour obtenir une réponse d'état permanent une fois que la résistance en série $R_S$ (303) du capteur a été déterminée ;

appliquer une tension de palier de compensation à une entrée non inverseuse (17) du circuit patch-clamp (100, 200, 360) ;

déterminer la constante de temps de la sortie (325) du circuit patch-clamp (100, 200, 360) à la tension de palier de compensation ; et

déterminer la capacité parasite d'entrée $C_P$ (301) du capteur à nanopore (302) à partir de la résistance en série $R_S$ (303) du capteur et de la constante de temps déterminée.

**14.** Procédé selon la revendications 13,

comprenant en outre l'étape de détermination d'une largeur d'impulsion de remise à zéro basée sur la capacité parasite d'entrée déterminée (301).

FIG. 1
(PRIOR ART)

FIG. 2

FIG. 3

200

16

$S_1$   12

$R_f$

18

AMP

+

10

17

104

D/A
Converter

202

**FIG. 4**

**FIG. 5**
**(Prior Art)**

**FIG. 6**

**FIG. 7**

**302**

**FIG. 8**

EP 2 734 839 B1

**FIG. 9**

22

_500_

START _502_

Activate headstage in transient mode _504_

Apply a step voltage to $V_{CMD}$ through DAC _506_

Measure time constant at $V_O$ _508_

Store it on a memory at PC _510_

Because $R_S$ is already known, $C_P$ can be calculated _512_

Based on the calculation, decide the reset pulse width ($T_t$) _514_

End stray capacitance compensation _516_

**FIG. 10**

**FIG. 11**

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5795782 A
- US 6015714 A
- US 6267872 B
- US 6627067 B
- US 6746594 B
- US 6428959 B
- US 6617113 B
- WO 2006028508 A

**Non-patent literature cited in the description**

- **WANG ; DUNBAR.** *Proc. 32nd Annual International Conference of the IEEE EMBS,* 2010, 2718-2721